# EUROPEAN PATENT APPLICATION

(11) **EP 4 039 332 A1**
(43) Date of publication of application: **10.08.2022**
(21) Application number: 22155248.2
(22) Date of filing: 04.02.2022
(51) Int. Cl.: A61P 29/00, A61P 19/00, A61K 36/28

(54) **METHOD OF EXTRACTION AND ENRICHMENT OF MONOGLYCOSYLATED FLAVONOIDS FROM CHRYSANTHELLUM INDICUM FOR THE TREATMENT OF INFLAMMATORY-BASED POLYARTHRITIC DISORDERS, FOR USE IN COSMETIC, NUTRACEUTICAL AND MEDICAL DEVICES AND PHARMACEUTICAL APPLICATIONS**

(30) Priority: 08.02.2021 IT 202100002735
(71) Applicant: Phytoitalia SRL, 20124 Milano (IT); Iontec S.a.r.l, 98000 Monaco (MC)
(72) Inventor: ABBIATI, Ezio, Bedizzole (BS) (IT); VOLPI, Nicola, Modena (IT)
(74) Representative: Rastelli, Franco

(57) **Abstract**

The invention relates to compositions and formulations rich in glycosylated flavonoids isolated from leaves, flowers, buds and other parts of *Chrysanthellum indicum* and to their structural characterization for the treatment of inflammatory-based polyarthritic pathologies. More particularly, the invention relates to compositions comprising a flavonoid isolated fraction extracted and purified by specific fractional precipitation and particularly rich in monoglycosylated derivatives capable of significantly reducing polyarthritic and inflammatory processes.

## Description

### FIELD OF THE INVENTION

The present invention refers to compositions and formulations rich in glycosylated flavonoids isolated from leaves, buds and other parts of *Chrysanthellum indicum* and to their structural characterization for the treatment of inflammatory-based polyarthritic disorders. More particularly, the invention relates to compositions comprising an isolated fraction of flavonoids extracted and purified by specific fractional precipitation and particularly rich in monoglycosylated derivatives capable of significantly reducing polyarthritic and inflammatory processes and active in finished products for use in cosmetics, nutraceutics, medical devices and pharmaceutical applications.

### BACKGROUND OF THE INVENTION

The Asteraceae family comprises approximately 100 genera and approximately 25,000-30,000 species. They are composite dicotyledons widespread in all latitudes in the temperate regions of North America, Europe, Africa and Asia. Belonging to the Asteraceae family is *Chrysanthellum,* of which 13 species are recognized, generally perennial herbs or shrubs with aromatic, lobed or serrated leaves. The varieties of *Chrysanthellum,* studied for their phytopharmaceutical applications generally in the form of aqueous or hydroalcoholic extracts, are the varieties *Chrysanthellum americanum vatke*, *Chrysanthellum indicum,* and *Chrysanthellum afro-americanum.*

*Chrysanthellum indicum* is a common variety especially in South American countries such as Peru and Bolivia, characterized by golden petals and for that reason called "golden chamomile". It is also known as Queen of the East and autumn flower and has been cultivated for over 2,000 years in Iran, where it is known as gul-e-daudi and is the second most important floriculture after the rose. It has been cultivated in China since the 15th century BC.

In Western culture, *Chrysanthellum* is seen as a symbol of luck, health, longevity and prosperity. In the East, *Chrysanthellum* is used in China and Japan as a food, drink and medicine for various diseases. *Chrysanthellum* tea is caffeine-free and is a great alternative to black tea and coffee. Chinese medicine uses *Chrysanthellum* tea for varicose veins, atherosclerosis, acne, flu, sore throat, fever, cold, high blood pressure, inflammation, type 2 diabetes.

Traditional Chinese Medicine (TCM) has been used for hundreds of years in China for the prevention and treatment of various diseases, the benefits claimed including antioxidant activities, improvement of the cardiovascular system and resistance to fatigue, reduction of serum lipid content. TCM uses yin-yang theory to explain the organization of structures, their physiology and pathological changes, as well as for diagnosis and treatment of the various diseases arising. Medicinal herbs are one of the main components of TCM. For this reason, in recent years the study of herbs used in medicine has grown enormously, using the flower of *Chrysanthellum indicum,* commonly called Ye-ju-hua in the Chinese pharmacopoeia, in addition to its leaves and stems.

The phytochemical and biological analysis of this plant has led to the identification of many secondary metabolites mainly represented by flavonoids, steroids and terpenoids. Camphor, borneol, camphene, α-pinene, p-cymene and 1,8-cineole are the main constituents of essential oils that have been extensively studied for their antimicrobial activity. Essential oils are a complex of secondary metabolites isolated from plants with high volatility and strong permeability. The importance of essential oils and plant extracts can be seen in their use in the pharmaceutical, food and cosmetic industries where they also have a significant economic impact. Furthermore, recent studies suggest that essential oils could be an alternative to synthetic fungicides due to their low toxicity and low residues.

*Chrysanthellum indicum* consists of 13 major compounds responsible for its many properties, such as acacetin-7-O-beta-D-glucopyranoside, luteolin, luteolin-7-O-beta-D-glucopyranoside, acacetin, acacetin-7-O-(6-O-alpha-L-rhamno pyranosyl)-beta-soforoside, 3-O-caffeoylquinic acid, syringaresinol-O-beta-D-glucopyranoside, 5,7-dihydroxychromone, uracil, hydroxybenzoic acid, 4-O-beta-D-glucopyranosiloxybenzoic acid, boscialina and blumenol A. *Chrysanthellum indicum* is composed of sesquiterpenes (54%) and monoterpenes (26%), but the percentages of the individual oils differ according to the technique used to analyze them, as well as the method of extraction.

The essential oils of *Chrysanthellum indicum* show antimicrobial and anti-infective activity, studied against *Staphylococcus aureus, Escherichia coli* and *Corynebacterium diphtheria. Chrysanthellum indicum* is also effective to reduce the toxicity of bleomycin, an antibiotic administered in combination with other cytostatic drugs for squamous cell carcinoma of the head and neck, Hodgkin's lymphoma, testicular cancer. *Chrysanthellum indicum,* in combination with bleomycin, can decrease levels of inflammatory cytokines (TNF-α and IL-6) in mouse tumors. In addition, it attenuates oxidizing enzymes such as myeloperoxidase. The transforming growth factor β (TGF-β1) also decreases following treatment with oleic extracts of *Chrysanthellum indicum.*

According to Chinese medicine and Ayurveda, the dried flowers of many *Chrysanthellum* plants are widely used for the treatment of common colds, fever, migraine, conjunctivitis, eye irritation, hypertension, inflammation, ulcerative colitis. In particular, the aqueous extract of *Chrysanthellum indicum* has proven to have powerful antioxidant effects, while the extracts obtained with methanol show inhibiting effects against xanthine oxidase in patients suffering from hyperuricemia and gout. In fact, xanthine oxidase is involved in the inflammatory process of gout, with the oxidation of hypoxanthine to xanthine and xanthine to uric acid. Xanthine oxidase represents the "gold standard" for plants used as medicines, since their extracts have few side effects, unlike drugs such as colchicine, steroids and NSAIDs that cause allergies, rush, kidney and liver dysfunction. Plants belonging to the Asteraceae family show 95% xanthine oxidase inhibitors and in particular *Chrysanthellum indicum* performs its inhibitory activity at 100 µg/mL. The compounds exhibiting this inhibitory activity could be the flavonoids luteolin and apigenin while various chemical compounds exhibit inhibitory effects against the production of nitric acid in macrophages. Polysaccharides are considered a fundamental part of Chinese medicine. In 1960 polysaccharides from various plants were discovered to possess antiviral properties. *Chrysanthellum indicum* was chosen for its soothing and detoxifying function of the liver to treat hepatitis caused in ducks by DHV-1 and DHV-E viruses. This disease affects chicks in the first three weeks of their life and is fatal. There is currently no cure. *Chrysanthellum indicum* polysaccharides (CIPS) are composed of rhamnose, arabinose, xylose, mannose, glucose, galactose and fructose. CIPS have effects on the immune system but little work has been done on their antimicrobial activity. Phosphorylation, post-translational modification of sugars, produces antiviral, antitumoral and antioxidant activity.

Several studies have been conducted in the cosmetic field to verify the anti-aging benefits in cosmetic formulations. *Chrysanthellum indicum* exhibits various pharmacological benefits both in vitro and in vivo and its organic extracts with solvents attenuate skin inflammation and atopic lesions in mice. It also acts on patients with moderate rosacea and is also used as an additive in cosmetics, although there are no studies into the effects on the skin of healthy patients.

In some experiments, the aqueous extract of *Chrysanthellum indicum* inhibits the activity of tyrosinase, a key enzyme in the catalysis of melanin biosynthesis. Responsible for the color of the skin, an excessive accumulation of melanin leads to hyperpigmentation, senile freckles, and melasma. Natural or synthetic tyrosinase inhibitors could be used as whiteners or anti-hyperpigmentation agents, due to their ability to suppress melanin production. Methanol extraction of *Chrysanthellum indicum* generates an anti-tyrosinase activity, similar to that of kojic acid, a lightening agent. The aqueous extract has a similar effect.

Luteolin, a flavone obtained from an aqueous extract, also exhibits strong anti-melanogenic activity, suggesting that luteolin directly and indirectly inhibits tyrosinase pathway 5. In particular, the O-glycosylation of flavonoids (7-*O-*glucoside, 7-O-galactoside, and 7-O-rutinoside), increases the inhibition of tyrosinase, the anti-HIV activity, the anti-rotavirus and anti-cholinesterase activity. In vitro, *Chrysanthellum indicum* extract inhibits tyrosinase activity and application of the cosmetic formulation containing 0.5% aqueous *Chrysanthellum indicum* extract reduces melanin levels in human volunteers. Nonetheless, long-term studies are needed to evaluate its true efficacy.

The major components of *Chrysanthellum indicum* are flavonoids, terpenoids and phenolic compounds. The flavonoids in the alcoholic extract of *Chrysanthellum indicum* exist in the form of glycosides which, however, have a low bioavailability due to their high polarity and poor absorption capacity. For this reason, several studies report that the treatment of plant extracts with enzymes capable of converting glucosides into aglycones increases bioavailability. For example, Viscozyme is an endo-beta-glucanase capable of extracting polyphenols and tannase serves to break the ester bond into various compounds. As reported above, glycosides have a low absorption capacity while aglycones produced by hydrolysis can instead be absorbed immediately. In many studies, plant enzymatic hydrolyzates have proven to have various antioxidant, anticoagulant and antiproliferative properties.

Adipogenesis and the accumulation of intracellular lipids regulate lipid metabolism and are involved in the development of obesity. The main flavonoids extracted from *Chrysanthellum indicum* are luteolin, apigenin, diosmetin and acacetin. *Chrysanthellum indicum* has proven effective in obese persons due to the presence of luteolin, apigenin and acacetin which are inhibitors of adipogenesis and lipid absorption. The hepatoprotective-hepatostimulating, anti-edematous, anti-inflammatory properties of *Chrysanthellum* have been useful in the production of patents aimed at improving health.

*Chrysanthellum indicum* has proven effective in the prevention and/or treatment of chronic inflammatory bowel diseases such as Crohn's disease and/or ulcerative colitis, often associated with overweight and obesity. It is known that patients affected by these pathologies have a reduction in *Bacteroides* and an increase in *Firmicutes* in the intestinal microbiota.

*Chrysanthellum indicum,* as single extract or in association with *Vaccinium myrtillus* and *Piper nigrum,* of *Piper aduncum* and/or *Piper longum,* may preferably be administered orally to humans and animals in the form of powder, gel, emulsion or liquid form. In vivo studies on mice fed a diet rich in lipids show an increase in Bacteroides and a decrease in Firmicutes in the intestinal microbiota, after taking a hydroalcoholic extract of a powder mixture obtained from the aerial parts of *Chrysanthellum indicum,* leaves of *Cynara scolymus,* fruits of *Vaccinium myrtillus* (3.7% of the composition), fruits of *Piper nigrum* (0.04% of the composition), leaves of Olea europaea (22.2% of the composition) and an excipient (silica) (patent no. FR3076997A1).

Numerous studies have focused on skin care, the skin being an organ ever more at the center of social life. Environmental pollution, a hectic lifestyle, stress, excessive exposure to sunlight, nutrition can create problems of redness, swelling, or itching through to true and proper allergic manifestations. Many Chinese companies have channeled their resources into the study of new formulations based on plant and root extracts with anti-inflammatory and antioxidant activity. The formulation of patent no. CN104083319A is nourishing and hydrating, without toxic effects or side effects, is easily absorbed and activates facial microcirculation and restores the tissue of skin fibers. In addition to *Chrysanthellum indicum* extract, there is also ginkgo biloba extract, Damask rose extract, *Punica granatum* extract and *Fagus sylvatica* extract.

Another invention is aimed precisely at sensitive skin, with the creation of a soothing cream with an anti-inflammatory effect based on *Chrysanthellum indicum* that repairs sensitive skin, licorice extract that protects from sunlight, camellia seed oil extract which gives elasticity to the skin (patent no. CN109010115A).

With a concentration of 0.003%, *Chrysanthellum indicum* extract allows keratinocytes damaged by UVB radiation to recover part of the initial ATP level compared to untreated damaged keratinocytes. It is therefore ideal for the creation of sun creams and after-sun creams (patent no. WO9820851A1).

Another patent concerns the creation of a tonic based on an extract of *Dendrobium, Chrysanthellum indicum*, cactus, ginseng root and honey with a calming and anti-inflammatory effect. It acts on irritations of the skin, hydrating it and maintaining the physiological balance of the skin without any allergic effect (patent no. CN109758381A).

Patent no. WO2016065688A1 concerns the creation of an anti-aging formulation in response to the increasingly pressing demand for cutting-edge cosmetics to counteract the onset of wrinkles and sagging facial features. The invention promises moisturizing and firming effects, thanks to collagen, it counteracts the onset of wrinkles caused by UV rays and free radicals by means of ceramides and tocopherols. Snail mucus, rich in allantoin, collagen and vitamins, helps cell renewal. *Chrysanthellum indicum* performs its antibacterial and anti-inflammatory function.

Patent no. WO2004006943A2 provides for the use of *Chrysanthellum indicum* for a preparation for topical use for the treatment of rosacea and erythrosis or erythro-couperosis. These skin conditions are characterized by redness of the face and upper chest area associated with capillary stasis and inflammatory reactions and an edematous state with or without apparent microvessels. In addition to the pain and discomfort caused by the inflammatory and edematous condition, the position on the face and upper chest area adds an unsightly component to the severity of this condition.

In rosacea, there is a rash characterized by facial erythema, telangiectasias and papulo-pustules, most often located in the nasolabial folds, cheekbones, nose and chin. Many dermatological preparations have been used in the treatment of these skin diseases, without any real efficacy. In the case of rosacea, drugs with antibacterial and anti-inflammatory activity, such as metronidazole, are also used. For this formulation, the *Chrysanthellum Indicum* extract used is obtained by implementing an extraction process conventionally used to treat plants. The average flavonoids content in the *Chrysanthellum* dry extract used is between 7 and 9%. The solutions used for preparation of the compositions according to the invention are 2% dry extract solutions.

*Chrysanthellum Indicum* extract can be used alone or in combination with other plant extracts having a beneficial effect on erythro-couperosis. Tests on patients followed for 12 weeks have shown that cream containing a *Chrysanthellum Indicum* extract allowed a significant efficacy and good tolerance to erythema and moderate rosacea to be obtained, with a notable improvement being observed in the severity of the erythema and skin damage, compared to a placebo. The compositions according to the invention also show a good skin tolerance and a marked effect on the degree of hydration with good persistence for 8 hours.

*Crocus sativum* is the plant from which comes saffron, whose properties range from the treatment of depression, to improvement of the metabolism and to anti-inflammatory action. The cosmetic and herbal industry uses this plant for the production of creams to counteract the cell damage that free radicals cause. The face mask of this patent provides for the use of *Crocus sativum*, *Chrysanthellum indicum* and hyaluronic acid with the aim of increasing the brightness of the face and increasing resistance to free radicals (patent no. EP3552598A1).

There are numerous patents based on herbs such as gangguk, macki and sechangpo. *Chrysanthellum indicum* has a detoxifying, analgesic and antibacterial activity, together with Portulaca oleracea, Acorus gramineus (plant belonging to the Acoracee family), Rumex japonica, Lonicera japonica Thunb, Artemisia vulgaris L., Rehmannia glutinosa Liboschitz var. purpurea Makino, ginseng, Poria cocos Wolf, Thea sinensis Linnaeus, Swertia japonica Makino, Polygonum multiflorum or Glycine max (patent no. KR20110060238A).

Another invention has as its target the reduction of body weight through the creation of an oral preparation based on *Chrysanthellum indicum* and horse chestnut capable of stimulating circulation and inhibiting the proliferation of adipocytes (patent no. JP2004359674A).

Increased body weight, obesity, increased LDL concentration are conditions that result from lifestyle and lead to an increase in the incidence of cardiovascular disease. Reducing LDL usually involves using statins along with other compounds such as nicotinic acid and polyunsaturated fatty acids. However, these drugs appear to have unwanted side effects, which is why the inventors sought a product that could satisfy the requests to improve the health of the individual, preventing annoying side effects. *Chrysanthellum indicum* together with artichoke extract *(Cynara scolymus)* and *Lycium barbarum* produce beneficial effects on lipid metabolism (patent no. WO2017068057A1).

*Chrysanthellum indicum* has also been used for intestinal disorders, as well as liver or gall bladder problems, kidney stones, gout. The hydroalcoholic preparation of the patent no. FR2096902A1 has a beneficial action in case of cystitis, alcohol-related cirrhosis and has a slightly positive action in chronic pancreatitis in association with conventional therapies. A patent has been proposed starting from the preparation of patent no. FR2096902A1 starting from an extraction and purification procedure with a reduced number of washes (2 washes against the 20 of the French patent), using an organic solvent such as dichloromethane and obtaining an average flavonoid/saponin ratio of 2/1. In vivo tests on animals have shown the absence of toxicity. The extract has anti-edematous properties and is particularly effective in cystinuria. Flavonoids and saponins are also effective on microcirculation, capillaries and also on platelet and erythrocyte aggregation. Furthermore, *Chrysanthellum indicum* present in the preparation (tablets, capsules, powders and syrups) also finds application in the treatment of dyslipidemias and above all of hypercholesterolemia and hyperlipidemia (patent no. EP0317453A1).

### SUMMARY OF THE INVENTION

It has now been found that an active fraction particularly effective from a pharmacological point of view in treating inflammatory-based polyarthritic pathologies essentially consisting of a high quantity of glycosylated flavonoids can be conveniently obtained by subjecting leaves, flowers, buds and other parts of *Chrysanthellum indicum* to a simple and convenient, specific fractional precipitation process.

This process has proven to be more efficient than the use of generic extracts based on solvents such as water, ethanol, methanol, butanol or mixtures consisting of high percentages, greater than 70%, of ethanol and lower percentages of water, generally less than 30%. The present process has proven to be more efficient than what is known in the state of the art as it surprisingly succeeds in extracting and purifying a large amount of monoglycosylated flavonoids, which constitute the active fraction capable of providing specific protection from inflammatory-based polyarthritic processes.

In fact, the fraction obtained, enriched in glycosylated flavonoids, in particular monoglycosylated, is particularly active in considerably and significantly decreasing inflammatory-based polyarthritic processes as evaluated in a specific, preclinical animal model.

The isolated fraction of the invention is characterized by a content of monoglycosylated flavonoids higher than 80% by weight and the remaining fraction of aglycone flavonoids lower than 20%.

The isolated fraction of the invention is characterized by a content of aglycone flavonoids which include in particular maritimetin in an amount greater than 2% by weight as a precursor of two of the main glycosylated derivatives, marein and maritimein, present in a concentration of not less than 30%.

In one embodiment, the invention provides an isolated *Chrysanthellum indicum* fraction enriched with monoglycosylated flavonoids in which the glycosidic fraction further comprises O-glucosides and O-glucuronides with the remaining fraction comprising aglycone flavonoids.

The remaining fraction of aglycone flavonoids of less than 20%, in addition to maritimetin, also contains isookanin, apigenin, chrysin, pinocembrin, galangin and caffeic acid.

The isolated fraction contains monoglycosylated flavonoids in a percentage greater than 80% which include, in addition to marein (2',3,3',4,4'-pentahydroxy-4'-glucosylchalcone) and maritimein (6-*O*-glycoside of maritimetin), also eriodictyol-7-O-glucoside, luteolin-7-O-glucoside and apigenin-7-O-glucuronide.

The isolated fraction comprising monoglycosylated flavonoids in a percentage greater than 80% with marein and maritimein greater than 30%, an aglycone fraction of less than 20% which includes maritimetin in a concentration greater than 2% and the remaining aglycone flavonoid fraction of less than 18%, is highly active in reducing polyarthritic pathologies as determined in a specific and widely used preclinical animal model.

The invention also relates to a process for the preparation of the active fraction from *Chrysanthellum indicum* comprising the steps of:
(a) drying the leaves, flowers, buds or other parts of the *Chrysanthellum indicum*;
(b) fragmenting the leaves, flowers, buds or other parts of the dried *Chrysanthellum indicum* using a manual or mechanical grinder capable of reducing the different parts into a fine powder consisting of particles of between 100 and 1,000 micrometers;
(c) treating the *Chrysanthellum indicum* with ethanol;
(d) subjecting the solution obtained to fractional precipitation with a specific amount of water so as to reach a specific organic phase/aqueous phase ratio;
(e) removal of the insoluble phase by filtration and/or centrifugation and recovery of the clear liquid phase;
(f) subjecting the solution obtained in the previous point to further fractional precipitation with an amount of water so as to reach a further specific organic phase/aqueous phase ratio;
(g) recovery of the insoluble phase by centrifugation and elimination of the liquid phase;
(h) the insoluble phase can be dried and used as such in finished preparations requiring solid material;
(i) alternatively, adding to the insoluble phase a 50/50 ethanol/water mixture to give a fraction rich in monoglycosylated flavonoids greater than 80% and aglycones lower than 20%.

The invention also provides pharmaceutical and nutraceutical compositions comprising the fraction obtained by the process mentioned above. The composition of the invention can be useful for a variety of therapeutic applications against inflammatory-based polyarthritic pathologies.

### BRIEF DESCRIPTION OF THE DRAWINGS

The invention will be described below in some preferred embodiments illustrated, by way of non-limiting example, in the figures of the attached tables, in which:
- Figure 1 illustrates an arthrogram obtained by administering 100 mg/kg of dry extract of *Chrysanthellum indicum* produced according to the present invention in a mouse model of adjuvant arthritis;
- Figure 2 illustrates the plasma levels of IL-1beta measured by administering 100 mg/kg of dry extract of *Chrysanthellum indicum* produced according to the present invention in a mouse model of adjuvant arthritis;
- Figure 3 illustrates the plasma levels of CRP measured by administering 100 mg/kg of dry extract of *Chrysanthellum indicum* produced according to the present invention in a mouse model of adjuvant arthritis;

- Figure 4 illustrates an arthrogram obtained by administering 100 mg/kg of dry extract of *Chrysanthellum indicum* produced according to the present invention in a mouse model of adjuvant arthritis;
- Figure 5 illustrates the plasma levels of IL-1beta measured by administering 100 mg/kg of dry extract of *Chrysanthellum indicum* produced according to the present invention in a mouse model of adjuvant arthritis;
- Figure 6 illustrates the plasma levels of IL-17A measured by administering 100 mg/kg of dry extract of *Chrysanthellum indicum* produced according to the present invention in a mouse model of adjuvant arthritis;
- Figure 7 illustrates the plasma levels of CRP measured by administering 100 mg/kg of dry extract of *Chrysanthellum indicum* produced according to the present invention in a mouse model of adjuvant arthritis.

### DETAILED DESCRIPTION OF THE PREFERRED EMBODIMENTS

As used herein, the term glycosylated flavonoids encompasses a very large heterogeneous family of compounds comprising a flavonoid linked to a saccharide portion consisting of one or more sugars linked by O-glycoside bonds. The nature of the flavonoid can be different depending on the type of flavonoid linked to the saccharide portion while the glycosidic part can be constituted by a single monosaccharide, the so-called monoglycosides, or by several saccharide units. In monoglycosides, the nature of the glycoside can be a simple monosaccharide or an acid derivative thereof on carbon 6 called glucuronic acid or glucuronide. The isolated active fraction of the invention contains monoglycosylated flavonoids consisting in particular of eriodictyol-7-*O-*glucoside, luteolin-7-O-glucoside, apigenin-7-O-glucuronide, marein chalcone (2',3,3',4,4'-pentahydroxy-4'-glucosylchalcone) and the maritimein aurone (6-*O-*glycoside of maritimetin).

As used herein, the term aglycone flavonoids encompasses a very large heterogeneous family of polyphenolic compounds consisting of variously substituted phenolic rings condensed together without bonds with monosaccharide or oligosaccharide sugars. The isolated active fraction of the invention contains various aglycone flavonoids in a concentration lower than 20% consisting specifically of maritimetin, precursor of mareima and maritimein, in amount greater than 2%. The remaining fraction of aglycone flavonoids in concentrations below 18% also consists of isookanin, apigenin, chrysin, pinocembrin, galangin and caffeic acid.

As used herein, the terms "amount", "concentration" and "percentage" refer to the content of the various glycosylated and non-aglycone flavonoid components, expressed as a percentage by weight of the individual biomolecules based on the total weight of the fraction of the present invention.

The method provides the steps of:
(a) drying of the leaves, flowers, buds or other parts of *Chrysanthellum indicum*;
(b) fragmentation of the leaves, flowers, buds or other parts of the dried *Chrysanthellum indicum* to obtain a fine powder consisting of particles of between 100 and 1,000 micrometers;
(c) treatment of *Chrysanthellum indicum* with ethanol;
(d) fractional precipitation by adding water to the ethanolic solution so as to achieve an organic phase/aqueous phase ratio of 80/20 v/v;
(e) decanting at low temperatures and filtration and/or centrifugation;
(f) removal of the insoluble phase;
(g) recovery of the clear liquid phase;
(h) further fractional precipitation by adding to the liquid phase an amount of water so as to reach an organic phase/aqueous phase ratio of 50/50 v/v;
(i) decanting at low temperatures and centrifugation;
(j) removal of the liquid phase;
(k) recovery of the precipitate;
(l) drying of the insoluble phase and removal of the residual solvent from the fraction obtained in step (k), by means of low temperature drying or rotary evaporation or equivalent methods, freeze-drying or a combination thereof;
(m) solubilization of the dried insoluble phase with a mixture of ethanol/water 50/50 v/v to give a fraction rich in monoglycosylated flavonoids greater than 80% and aglycones lower than 20%.

The fraction obtained in step (k), (I) or (m) is substantially rich in monoglycosylated flavonoids consisting in particular of eriodictyol-7-O-glucoside, luteolin-7-O-glucoside, apigenin-7-O-glucuronide, marein chalcone (2',3,3',4,4'-pentahydroxy-4'-glucosylchalcone) and maritimein aurone (6-*O*-glycoside of maritimetin) in a concentration greater than 80%.

The fraction obtained in step (k), (I) or (m) contains, among the monoglycosylated flavonoids, marein and maritimein in a percentage greater than 30%.

The fraction obtained in step (k), (I) or (m) contains various aglycone flavonoids in a concentration lower than 20%.

The fraction obtained in step (k), (I) or (m) contains maritimein among the aglycone flavonoids, in an amount greater than 2%.

The fraction obtained in step (k), (I) or (m), among the aglycone flavonoids, in an amount lower than 18%, also contains isookanin, apigenin, chrysin, pinocembrin, galangin and caffeic acid.

The composition of the fraction obtained in step (k), (I) or (m) is substantially free of flavonoids and derivatives soluble in a mixed ethanol/water environment lower than 80/20 v/v and greater than 50/50 v/v.

Other parts of the plant such as the stem and roots can be used as an alternative or together with the leaves, flowers and buds of *Chrysanthellum indicum.*

The process of the invention is disclosed in detail below in this document.
STAGE 1. Preparation of the solution of *Chrysanthellum indicum* raw material in an appropriate solvent
   i. The *Chrysanthellum indicum* raw material, in particular leaves, flowers and buds but also other parts such as stem and roots, are dried in the oven at low temperatures, between 30 and 50°C, preferably 40°C. The drying is carried out in conditions of low humidity, between 20 and 50% relative humidity, preferably between 30 and 35%, for a period of not less than 20 hours, preferably for a time between 20 and 30 hours.
   ii. The dried *Chrysanthellum indicum* raw material from point i. is crushed using preferably industrial crushers to obtain a fine powder consisting of particles with dimensions ranging from 100 to 1,000 micrometers, preferably from 400 to 600 micrometers.
   iii. The material of point ii. is sieved on sieves provided with porosity lower than 1,000 micrometers to recover the sieved material with dimensions lower than 1,000 micrometers. The material remaining on the sieve is again crushed and sieved. The cycle is repeated until most of the *Chrysanthellum indicum* raw material has particle size below 1,000 micrometers.
   iv. The material of point iii. is dissolved in 100% ethanol.
   v. The *Chrysanthellum indicum* raw material is dissolved in the solvent of point iv. at a weight/volume concentration from 10 mg/mL to 50 mg/mL, typically at a concentration between 25 and 30 mg/mL.
   vi. The *Chrysanthellum indicum* solution of point v. is mixed under stirring at a temperature from 20°C to 30°C, preferably from 23°C to 26°C. The solution is stirred for a period of 5 to 10 hours, preferably from 6 to 8 hours.
   vii. The *Chrysanthellum indicum* raw material is subsequently treated in an ultrasonic bath at a controlled temperature between 25 and 40°C, preferably between 30 and 35°C, with a power from 50 to 100 Watts, preferably 70 Watts, for a period of time of between 5 and 15 hours, preferably between 5 and 10 hours.
   viii. The solution of point vii. is centrifuged to remove the insoluble material at between 5,000 and 10,000 rpm, preferably 8,000 rpm, at a temperature from 20°C to 30°C, preferably 25°C, for 20 to 30 minutes, preferably 25 min. The solution of point vii. can be filtered or further filtered after centrifugation on cellulose filters at a temperature from 25°C to 30°C, preferably 28°C, this slightly higher temperature favoring filtration by gravity.
STAGE 2. First fractional precipitation
   i. To the ethanolic solution of *Chrysanthellum indicum* of point viii. an adequate volume of distilled water is added so as to reach an ethanol/water ratio of between 78/22 and 82/18 v/v, preferably 80/20 v/v.
   ii. The solution of point i. is decanted at low temperatures, between 2 and 6°C, preferably at 4°C, for a time of between 4 and 12 hours, preferably between 7 and 10 hours, preferably for 8 hours.
   iii. The liquid part of the solution without the precipitate is recovered by suction or filtration on cellulose filters at a temperature between 25°C and 30°C, preferably 28°C.
   iv. The solution of point iii. is then centrifuged to remove any insoluble microparticulate still present in solution by centrifugation between 8,000 and 12,000 rpm, preferably 10,000 rpm, at a temperature from 16°C to 25°C, preferably 22°C, for a time of between 20 and 40 minutes, preferably 30 minutes.
   v. The solution centrifuged in step iv. and free from any microparticulate insoluble in ethanol/water 80/20 v/v is recovered by pouring it into a new container.
   vi. The insoluble part precipitated by decantation in point ii. and obtained by centrifugation at point v. is eliminated.
STAGE 3. Second fractional precipitation
   i. To the mixed solution ethanol/water 80/20 v/v of *Chrysanthellum indicum* from point v. an adequate volume of distilled water is added so as to reach an ethanol/water ratio of between 46/54 and 52/48 v/v, preferably 50/50 v/v.
   ii. The solution of point i. is decanted at low temperatures, between -20 and -5°C, preferably at -16°C, for a time of between 10 and 20 hours, preferably between 13 and 18 hours, preferably for 15 hours.
   iii. A precipitate and a liquid phase are formed.
   iv. The liquid phase of point iii. is recovered by suction and eliminated.
   v. The precipitate of point iii. without the liquid phase is recovered and centrifuged to completely eliminate the liquid phase consisting of ethanol/water 50/50 v/v still present in a limited volume. Centrifugation takes place between 12,000 and 20,000 rpm, preferably 15,000 rpm, at a temperature of 4°C to 15°C, preferably 10°C, for a time of between 30 and 60 minutes, preferably 50 minutes.
   vi. The precipitate obtained from the centrifugation in the previous point v., free from the liquid phase and insoluble in ethanol/water 50/50 v/v is recovered.
STAGE 4. Drying of *Chrysanthellum indicum* extract rich in glycosylated flavonoids
   i. The precipitate obtained from centrifugation vi. and insoluble in ethanol/water 50/50 v/v is dried by evaporation of the residual solvent at low temperatures, between 20 and 40°C, preferably at 30°C, since higher temperatures induce the breaking of the O-glycoside bond with the release of the flavin and glycosidic part, resulting in loss of the anti-polyarthritic activity. In a preferred embodiment, drying using rotary evaporators at low temperature, not exceeding 35°C, or freeze-drying, or a combination thereof, is used.
   ii. The dry extract obtained in the previous point i., which constitutes the glycosylated active part object of the present invention, can be used as is or solubilized in a 50/50 v/v ethanol/water mixture to produce active liquid products.

The two products, i.e. the dry product of point i. and the product in ethanol/water 50/50 v/v solution of previous point ii., were tested by HPLC-MS (single quadrupole electrospray ionization mass) for the presence and content of glycosylated flavonoids.

HPLC-MS, by using reverse phase analytical columns, is able to separate glycosylated and aglycone flavonoids on the basis of their structure and hydrophobicity and detected by MS. Furthermore, the MS and mass tandem are able to identify the different molecules based on their mass values and fragmentation patterns.

As exhaustively described in the examples, the two fractions obtained by double fractional precipitation, i.e. the dry fraction and the liquid fraction with added ethanol/water 50/50, contain monoglycosylated flavonoids in concentrations greater than 80% by weight and aglycone flavonoids in amount lower than 20% by weight. The monoglycosylated portion greater than 80% is constituted by marein, maritimein, eriodictyol-7-*O*-glucoside, luteolin-7-*O-*glucoside and apigenin-7-*O*-glucuronide. The two main monoglycosylated flavonoids, marein and maritimein, are present in amounts greater than 30%.

On the other hand, the aglycone flavonoid fraction lower than 20% has a maritimetin content higher than 2% by weight as a precursor of the two main glycosylated derivatives, marein and maritimein. The remaining fraction of aglycone flavonoids lower than 18%, in addition to maritimetin also contains isookanin, apigenin, chrysin, pinocembrin, galangin and caffeic acid.

As exhaustively described in the examples, the two fractions obtained by double fractional precipitation, the dry fraction and the liquid fraction with added ethanol/water 50/50, are highly active in reducing inflammatory-based polyarthritic processes as tested in a specific preclinical animal model for these pathologies.

The insoluble part of point vi. obtained in STAGE 2 and precipitated by centrifugation and the liquid phase of point iv. obtained in STAGE 3 by centrifugation, are also analyzed by HPLC-MS for the presence and content of glycosylated flavonoids, demonstrating the absence or a very low content thereof.

The fractions obtained by the process described above can be suitably formulated, in admixture with an acceptable vehicle in a form suitable for oral, topical, parenteral or transdermal administration, for cosmetic, dermatological, pharmaceutical or nutraceutical applications.

A topical formulation may be suitable for dermal, vaginal, rectal, inhalation, intranasal, ocular, auricular, or buccal administration. Examples of suitable vehicles include a mineral oil or a vegetable oil or a combination thereof. The carrier may further comprise at least one wax, such as beeswax, vegetable waxes, sugar cane waxes, mineral waxes and synthetic waxes.

Examples of suitable forms include capsules, tablets, liposomes, suppositories, suspensions, ointments, creams, lotions, solutions, emulsions, nanoemulsions, microemulsions, films, cements, powders, glues, aerosols, or sprays.

Mouse models of osteoarthritis and rheumatoid arthritis are useful tools for the study of these pathologies. Adjuvant arthritis is one of the most widely used polyarthritis models to test numerous preclinical and clinical antiarthritic agents as well as different possible therapeutic approaches. The hallmarks of this model are robust polyarticular inflammation, marked bone resorption, and periosteal bone proliferation. Cartilage destruction occurs but is disproportionately mild compared to the inflammation and bone destruction that occur. For this, adjuvant arthritis in mouse models has also been extensively used for pharmaceutical testing of anti-inflammatory agents.

The fractions of the invention are active against inflammatory-based polyarthritic processes and are therefore useful for the treatment of all acute and/or chronic arthritic processes generated by inflammatory conditions. The fractions of the invention are active against rheumatic fever, against osteoarthritis and arthritis, rheumatoid arthritis and other degenerative diseases of the bones and connective tissue, as well as in the treatment of amyloidosis, psoriatic arthritis and lupus erythematosus. The fractions and compositions of the invention are also useful for the treatment of inflammatory-based pathologies that develop as a result of polyarthritic processes, such as inflammation of the mucous membranes and the skin, such as gastric and duodenal inflammation and peptic ulcer, active chronic gastritis and gastric atrophy, but also in the treatment of skin disorders, to induce tissue repair and wound healing, for eczema, psoriasis, seborrheic keratosis and ulcers.

The therapeutically effective amount of the fractions of the invention can vary according to the recipient subject, the route and the frequency of administration. In general, the amount may conveniently be in the range of about 0.1% to about 50% w/w with respect to the total weight of the composition.

The pharmaceutical or nutraceutical compositions of the invention can be produced by conventional mixing, granulation, softgel encapsulation, dissolution, extraction or freeze-drying processes, and solutions.

The invention is described in greater detail in the following examples.

### EXAMPLE 1

*Chrysanthellum indicum* leaves and flowers are dried in an oven at 40°C at a relative humidity of 30% for 24 hours. The dry material is crushed by manual crushing and sieving on sieves smaller than 1,000 micrometers, a powder is obtained consisting of particles of between 200 and 1,000 micrometers. The cycle is repeated until most of the *Chrysanthellum indicum* leaves and flowers are recovered following sieving with particle sizes below 1,000 micrometers.

The dry material reduced to dimensions below 1,000 micrometers is solubilized in 100% ethanol at a concentration of 25 mg/mL. Specifically, 1,000 mg of *Chrysanthellum indicum* dry material in 40 mL of ethanol are mixed under continuous stirring at room temperature of 24°C for 8 hours. After 8 hours of continuous mixing, the solution is transferred to an ultrasonic bath and treated at 35°C with a power of 75 Watt for further 8 hours.

The solution obtained is centrifuged at 8,000 rpm, at 25°C, for 30 minutes and subsequently filtered by gravity on a cellulose filter at a temperature of 30°C.

To the 40 mL ethanolic solution of leaves and flowers of *Chrysanthellum indicum,* 8 mL of distilled water is added to obtain an ethanol/water ratio of 80/20 v/v. The resulting solution is left in the fridge at 4°C for 10 hours.

The liquid phase of the previous solution is recovered by filtration on nitrocellulose filters at a temperature of 30°C while the decanted insoluble material is removed. The solution is then further centrifuged to remove any insoluble microparticulate, centrifuging at 10,000 rpm at 25°C for 30 min. At this point in the process, the solution of *Chrysanthellum indicum* flowers and leaves in ethanol/water 80/20 v/v free from any insoluble microparticulate is recovered by pouring it into a new container. The insoluble part obtained by centrifugation is removed.

To the previously obtained *Chrysanthellum indicum* ethanol/water 80/20 v/v solution, 32 mL of distilled water is added in order to reach the ethanol/water ratio of 50/50 v/v. The solution obtained is left to decant in a freezer at -18°C. After 16 hours, the liquid part is removed by suction using an aspirator while the decanted insoluble part is centrifuged at 15,000 rpm at 10°C for 50 min. The precipitate obtained from centrifugation and free from any further possible liquid phase is recovered.

The solid part obtained by centrifugation as a result of its insolubility in ethanol/water 50/50 v/v is dried by evaporation of the residual solvent in an oven at 30°C.

280 mg of the fraction of the invention are obtained, equal to a recovery of 28%.

20 mg of the fraction of the invention are solubilized in 1 mL of ethanol/water 50/50 v/v and 10 microL are injected into a Jasco HPLC system equipped with a single quadrupole mass detector from Waters and the different flavonoid species are separated on a Discovery-C18 column from Sigma-Aldrich using a gradient consisting of ammonium acetate and formic acid.

Table 1 shows the biomolecular species present in the fraction of the invention and identified and quantized by the MS detector.

**Table 1. Molecular species and their amounts present in the dry fraction of Chrysanthellum indicum obtained by means of the present invention in Example 1 and identified and quantized by HPLC-MS.**

| **Peak in HPLC - Molecular Species** | **% w/w** |
|---|---|
| Caffeic Acid | 9.6 |
| Marein | 15.3 |
| Maritimein | 17.0 |
| Eriodictyol-7-O-Glucoside | 14.2 |
| Luteolin-7-O-Glucoside | 9.1 |
| Apigenin-7-O-Glucuronide | 27.4 |
| Isookanin | 1.3 |
| Maritimetin | 3.2 |
| Apigenin | 1.3 |
| Chrysin | 0.2 |
| Pinocembrin | 0.6 |
| Galangin | 0.8 |

As is evident from Table 1, the dry fraction of the present invention to which ethanol/water 50/50 has been added contains monoglycosylated flavonoids in a concentration of 83.0% by weight and aglycone flavonoids in an amount of 17.0% by weight. The 80% monoglycosylated portion is made up of marein, maritimein, eriodictyol-7-*O*-glucoside, luteolin-7-*O*-glucoside and apigenin-7-*O-*glucuronide. The two main monoglycosylated flavonoids, marein and maritimein, are present in amount of 32.3%.

On the other hand, the fraction of aglycone flavonoids of 17.0% has a maritimetin content of 3.2% by weight as a precursor of the two main glycosylated derivatives, marein and maritimein. The remaining fraction of aglycone flavonoids contains isookanin, apigenin, chrysin, pinocembrin, galangin and caffeic acid.

The dry fraction of the present invention is highly active in reducing inflammatory-based polyarthritic processes as tested in the preclinical animal model specific for this pathology, the mouse model of adjuvant arthritis.

Male Lewis rats, weighing 160-180 g, were used. The rats had free access to a standard pelleted diet and tap water. The facilities for animals were compliant with the European Convention for the Protection of Vertebrate Animals used for experimental purposes. Adjuvant arthritis was induced by a single intradermal injection of heat-inactivated *Mycobacterium butyricum* in the incomplete Freund's adjuvant (Difco Laboratories, Detroit, MI, USA). The injection was performed near the base of the tail. The experiments included healthy animals (HC), untreated arthritic animals (AA), and arthritic animals given 100 mg/kg body weight of the dry fraction of the present invention per day until the end of the experiment (day 28).

The arthrogram (or arthritic score) was measured as the sum of the individual scores relating to the volume of the hind leg (ml, max. 8 points), the diameter of the forelimb (mm, max. 5 points) and the diameter of the crust developed at the application site of *Mycobacterium butyricum*, measured parallel to the vertebral column (mm, max. 5 points) for each animal.

The determination of the pro-inflammatory interleukin IL-1beta and of the plasma C-reactive protein (PCR) were carried out by the ELISA technique using specific analytical kits.

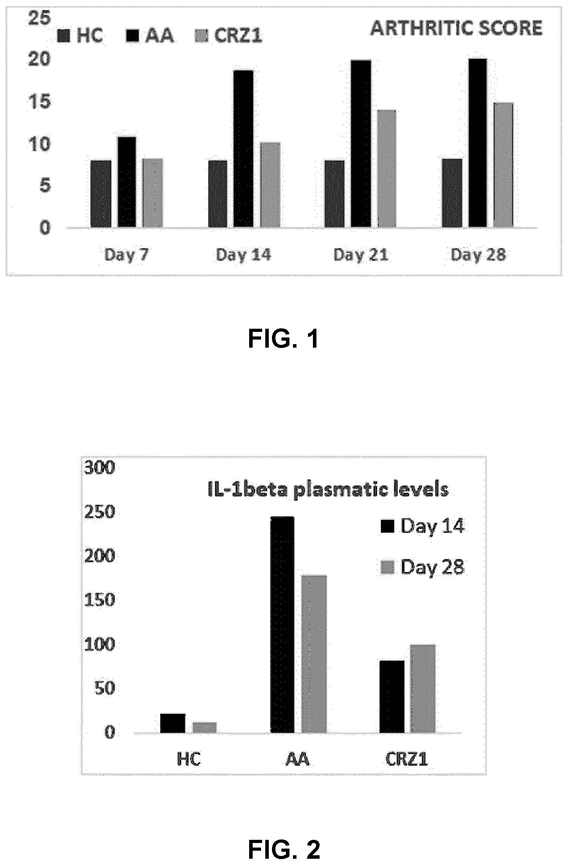

As clearly illustrated in Figure 1, the dry fraction of the present invention significantly reduces the arthritic score and therefore the incidence of the polyarthritic condition already after 14 days of treatment (-94.6%, p<0.00001), then continuing to be active after 21 days (-30.0%, p<0.01) and 28 days (-26.4%, p<0.05). As is evident from Figure 1, the extract (CRZ1) of the present example reduces the condition of polyarthritis (AA) induced in healthy animals (HC).

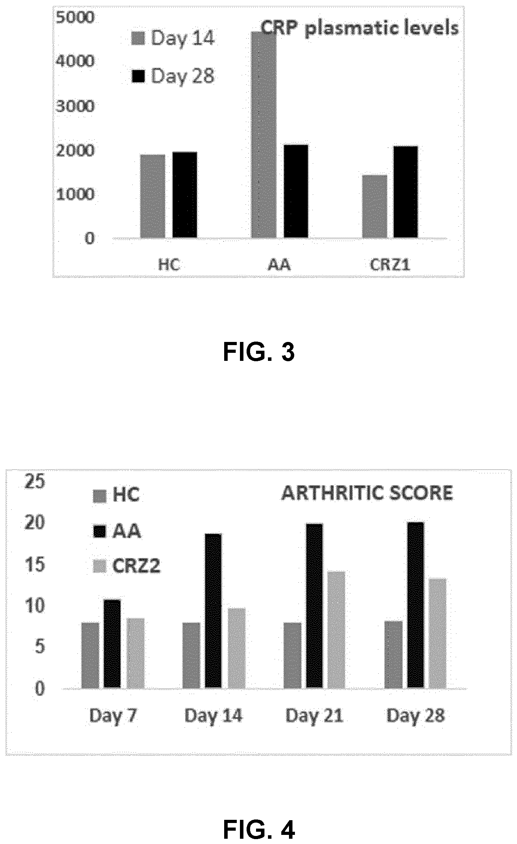

As is clearly evident in Figures 2 and 3, the dry fraction of the present invention has a strong anti-inflammatory activity, significantly reducing the plasma levels of IL-1beta and of the C-reactive protein, indicator of systemic inflammation. As is evident from Figure 2, the extract (CRZ1) of the present example reduces the levels of IL-1beta (-66.7%, p<0.05 after 14 days and -43.8%, p<0.05 after 28 days of treatment) in healthy animals (HC) in which adjuvant arthritis (AA) is induced. As is evident from Figure 3, the extract (CRZ1) of the present example reduces CRP levels (-69.3%, p<0.005 after 14 days of treatment) in healthy animals (HC) in which adjuvant arthritis (AA) is induced. As is evident, on day 28 the systemic inflammation is no longer present thanks to the anti-inflammatory effect of the dry extract of *Chrysanthellum indicum* of the present invention.

In conclusion, the dry fraction obtained from the present process of double and selective fractional precipitation contains monoglycosylated flavonoids in a concentration greater than 80% by weight and aglycone flavonoids in an amount of less than 20% by weight. The monoglycosylated portion greater than 80% is constituted by marein, maritimein, eriodictyol-7-*O*-glucoside, luteolin-7-*O-*glucoside and apigenin-7-*O*-glucuronide. The two main monoglycosylated flavonoids, marein and maritimein, are present in amounts greater than 30%. The aglycone flavonoid fraction lower than 20% has a maritimetin content greater than 2% by weight. The remaining fraction of aglycone flavonoids lower than 20%, in addition to maritimetin also contains isookanin, apigenin, chrysin, pinocembrin, galangin and caffeic acid.

Furthermore, the dry fraction of the present invention is highly active in reducing inflammatory-based polyarthritic processes as tested in the preclinical animal model specific for this pathology, the mouse model of adjuvant arthritis, as it determines a significant reduction of the arthritic score of one of the main pro-inflammatory interleukins (IL-1beta) and C-reactive protein, indicator of systemic and diffuse inflammation.

### EXAMPLE 2

Buds of *Chrysanthellum indicum* are dried in the air at an ambient temperature of 38°C and a relative humidity of 40% for 30 hours. The dry material is crushed by means of an electric shredder and sieved on sieves smaller than 1,000 micrometers. A powder is obtained consisting of particles with dimensions ranging from 500 to 1,000 micrometers. The cycle is repeated until most of the *Chrysanthellum indicum* buds are recovered following sieving with particle sizes between 500 and 1,000 micrometers.

The dry material, reduced to dimensions lower than 1,000 micrometers, is solubilized in 100% ethanol at a concentration of 40 mg/mL. Specifically, 3,000 mg of *Chrysanthellum indicum* dry material in 75 mL of ethanol are mixed under continuous stirring at room temperature of 27°C for 10 hours. After 10 hours of continuous mixing, the solution is transferred to an ultrasonic bath and treated at 30°C with a power of 100 Watt for a further 12 hours.

The solution obtained is centrifuged at 10,000 rpm, at 22°C, for 30 minutes and subsequently filtered by gravity on a cellulose filter at a temperature of 30°C.

15 mL of distilled water is added to the ethanolic solution of *Chrysanthellum indicum* buds with a volume of 75 mL to obtain an ethanol/water ratio of 80/20 v/v. The resulting solution is left in the fridge at 4°C for 12 hours.

After 12 hours of decantation, the liquid phase of the previous solution is recovered by filtration on nitrocellulose filters at a temperature of 30°C while the decanted insoluble material is removed. The solution is then further centrifuged to remove any insoluble microparticulate, centrifuging at 8,000 rpm at 22°C for 40 min. At this point in the process, the solution of *Chrysanthellum indicum* buds in ethanol/water 80/20 v/v free from any insoluble microparticulate is recovered by pouring it into a new container. The insoluble part obtained by centrifugation is removed.

To the previously obtained *Chrysanthellum indicum* ethanol/water 80/20 v/v solution, 60 mL of distilled water is added so as to reach a total volume of 150 mL of solution, of which 75 consisting of ethanol and 75 of water, for an ethanol/water ratio of 50/50 v/v. The solution obtained is left to decant in a freezer at -15°C. After 20 hours, the liquid part is removed by suction using an aspirator while the decanted insoluble part is centrifuged at 12,000 rpm at 5°C for 45 min. The precipitate obtained from centrifugation and free from further possible liquid phase is recovered.

The solid part obtained by centrifugation as a result of its insolubility in ethanol/water 50/50 v/v is dried by evaporation of the residual solvent in an oven at 25°C and subsequent freeze-drying.

750 mg of the fraction of the invention are obtained, equal to a recovery of 25%.

20 mg of the fraction of the invention are solubilized in 1 mL of ethanol/water 50/50 v/v and 10 microL are injected into a Jasco HPLC system equipped with a single quadrupole mass detector from Waters and the different flavonoid species are separated on a Discovery-C18 column from Sigma-Aldrich using a gradient consisting of ammonium acetate and formic acid.

Table 2 shows the biomolecular species present in the fraction of the invention and identified and quantized by the MS detector.

**Table 2. Molecular species and their amounts present in the dry fraction of Chrysanthellum indicum obtained by the present invention in Example 2 and identified and quantized by HPLC-MS.**

| **Peak in HPLC - Molecular Species** | **% w/w** |
|---|---|
| Caffeic Acid | 6.5 |
| Marein | 17.2 |
| Maritimein | 17.4 |
| Eriodictyol-7-O-Glucoside | 17.7 |
| Luteolin-7-O-Glucoside | 7.5 |
| Apigenin-7-O-Glucuronide | 24.9 |
| Isookanin | 1.7 |
| Maritimetin | 2.7 |
| Apigenin | 1.9 |
| Chrysin | 0.5 |
| Pinocembrin | 1.0 |
| Galangin | 1.0 |

As is evident from Table 2, the dry fraction with added ethanol/water 50/50 contains monoglycosylated flavonoids in a concentration of 84.7% by weight and aglycone flavonoids in an amount lower than 15.3% by weight. The monoglycosylated portion greater than 80% is constituted by marein, maritimein, eriodictyol-7-O-glucoside, luteolin-7-O-glucoside and apigenin-7-O-glucuronide. The two main monoglycosylated flavonoids, marein and maritimein, are present in amount of 34.6%.

On the other hand, the fraction of aglycone flavonoids of 15.3% has a maritimetin content of 2.7% by weight as a precursor of the two main glycosylated derivatives, marein and maritimein. The remaining fraction of aglycone flavonoids contains isookanin, apigenin, chrysin, pinocembrin, galangin and caffeic acid.

This second dry fraction of the present invention is also highly active in reducing inflammatory-based polyarthritic processes as tested in the preclinical animal model specific for this pathology, the mouse model of adjuvant arthritis. The data of the arthritic score, the levels of pro-inflammatory interleukins IL-1beta and IL-17A, and plasma C-reactive protein (PCR) are reported below.

Determination of the pro-inflammatory interleukins IL-1beta and IL-17A, and of the plasma C-reactive protein (PCR) was carried out by the ELISA technique using specific analytical kits.

As clearly illustrated in Figure 4, the dry fraction of the present invention in Example 2 significantly reduces the arthritic score and therefore the incidence of the polyarthritic condition already after 14 days of treatment (-48.4%, p<0.005), continuing then to be active after 21 days (-29.5%, p<0.005) and 28 days (-33.3%, p<0.005). As is evident from Figure 4, the extract (CRZ2) of the present example reduces the condition of polyarthritis (AA) induced in healthy animals (HC).

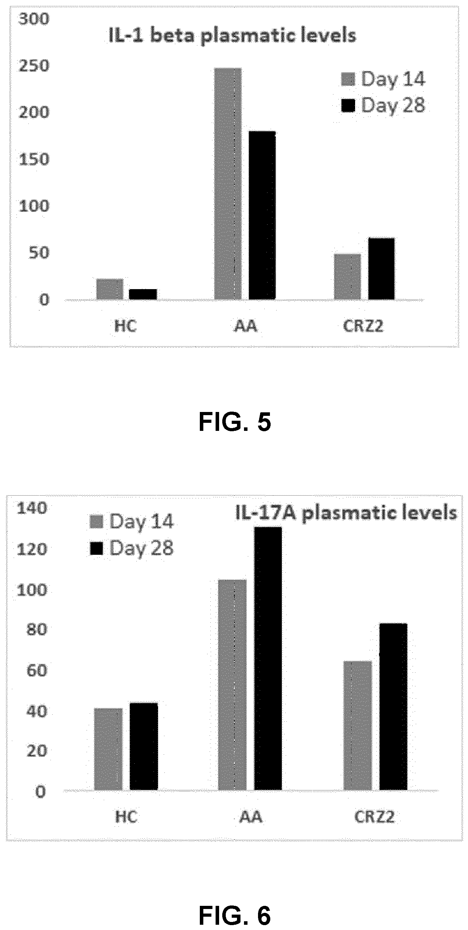

As is clearly evident in Figures 5 and 6, the dry fraction of the present invention exhibits a strong anti-inflammatory activity, significantly reducing the plasma levels of IL-1beta and IL-17A. As is evident from Figure 5, the extract (CRZ2) of the present example reduces the levels of IL-1beta (-80.5%, p<0.01 after 14 days and -63.3%, p<0.05 after 28 days of treatment) in healthy animals (HC) in which adjuvant arthritis (AA) is induced. As is evident from Figure 6, the extract (CRZ2) of the present example reduces the levels of IL-17A (-38.4%, p<0.005 after 14 days and -36.7%, p<0.05 after 28 days of treatment) in healthy animals (HC) in which adjuvant arthritis (AA) is induced.

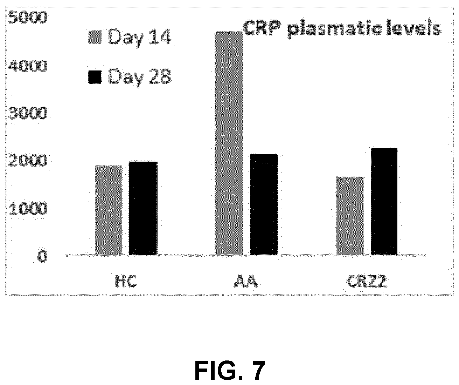

As shown in Figure 7, the dry fraction of the present invention also exhibits a strong anti-inflammatory activity, significantly reducing plasma levels of C-reactive protein, indicator of systemic inflammation. As is evident from Figure 7, the extract (CRZ2) of the present example reduces CRP levels (-64.3%, p<0.005 after 14 days of treatment) in healthy animals (HC) in which adjuvant arthritis (AA) is induced. As is evident, on day 28 the systemic inflammation is no longer present thanks to the anti-inflammatory effect of the dry extract of *Chrysanthellum indicum* of the present invention.

In conclusion, the second dry fraction obtained by the present process of double and selective fractional precipitation also contains monoglycosylated flavonoids in a concentration greater than 80% by weight and aglycone flavonoids in amount lower than 20% by weight. The monoglycosylated portion greater than 80% is constituted by marein, maritimein, eriodictyol-7-O-glucoside, luteolin-7-O-glucoside and apigenin-7-O-glucuronide. The two main monoglycosylated flavonoids, marein and maritimein, are present in amounts greater than 30%. The aglycone flavonoid fraction lower than 20% has a maritimetin content greater than 2% by weight. The remaining fraction of aglycone flavonoids lower than 20% contains isookanin, apigenin, chrysin, pinocembrin, galangin and caffeic acid.

Furthermore, this second dry fraction of the present invention is also highly active in reducing inflammatory-based polyarthritic processes as tested in the preclinical animal model specific for this pathology, the mouse model of adjuvant arthritis, as it causes a significant reduction of the arthritic score, of two of the main pro-inflammatory interleukins (IL-1beta and IL-17A) and of the C-reactive protein, indicator of systemic and diffuse inflammation.

## Claims

1. A fraction isolated from *Chrysanthellum indicum* in which the content of monoglycosylated flavonoids exceeds 80% by weight and aglycone flavonoids in amount lower than 20% by weight, in which said monoglycosylated portion comprises marein, maritimein and maritimetin.

2. The isolated fraction according to claim 1, in which the monoglycosylated portion greater than 80% further comprises eriodictyol-7-*O*-glucoside, luteolin-7-*O*-glucoside and apigenin-7-*O*-glucuronide.

3. The isolated fraction according to claim 2, in which the two main monoglycosylated flavonoids, marein and maritimein, are present in amounts greater than 30% by weight.

4. The isolated fraction according to claim 1, in which the portion of aglycone flavonoids lower than 20% also contains isookanin, apigenin, chrysin, pinocembrin, galangin and caffeic acid.

5. The isolated fraction according to claim 4, in which the portion of aglycone flavonoids lower than 20% contains maritimetin in an amount greater than 2% by weight.

6. The isolated fraction according to claim 1 active in reducing inflammatory-based polyarthritic processes.

7. The isolated fraction according to claim 6 active in the treatment of all acute and/or chronic arthritic processes caused by inflammatory conditions.

8. The isolated fraction according to claim 6 active in the treatment of inflammatory-based pathologies.

9. The isolated fraction according to claim 6 active in the treatment of degenerative diseases of the bones and connective tissue.

10. The isolated fraction according to claim 1 obtained from the whole plant of *Chrysanthellum indicum* or from selected parts thereof.

11. A process for the preparation of the fraction rich in specific monoglycosylated and aglycone flavonoids according to claims 1 to 5, comprising the steps of:
(a) drying and fragmenting the parts of *Chrysanthellum indicum* to obtain a fine powder consisting of particles with size smaller than 1,000 micrometers;
(b) treating *Chrysanthellum indicum* with ethanol;
(c) subjecting the solution obtained to a first fractional precipitation by adding water so as to reach an ethanol organic phase/aqueous phase ratio of 80/20 v/v;
(d) subjecting the solution obtained to decantation at low temperatures and subsequent filtration and/or centrifugation;
(e) recovery of the clear liquid phase and removal of the insoluble phase;
(f) subjecting the liquid phase obtained to a second fractional precipitation by adding an amount of water so as to reach an ethanol organic phase/aqueous phase ratio of 50/50 v/v;
(g) subjecting the solution obtained to decantation at low temperatures and subsequent centrifugation;
(h) recovery of the precipitate and removal of the liquid phase;
(i) subjecting the insoluble phase obtained to drying and removal of the residual solvent.

12. Process according to claim 11, in which the residual solvent in step (i) is removed by evaporation, freeze-drying, or a combination thereof.

13. Pharmaceutical, cosmetic or nutraceutical composition comprising the fractions according to claims 1 to 5.

14. Composition according to claim 13, in the form of capsules, tablets, suppositories, suspensions, ointments, creams, lotions, solutions, emulsions, films, powders, glues, aerosols, sprays.
